# EUROPÄISCHE PATENTSCHRIFT

(11) **EP 1 025 081 B1**
(45) Veröffentlichungstag und Bekanntmachung des Hinweises auf die Patenterteilung: **13.03.2002**
(21) Anmeldenummer: 98954397.0
(22) Anmeldetag: 12.10.1998
(51) Int. Cl.: C07C 381/02, C07C 319/06, C07C 319/14, C07C 321/26, C07C 321/28, C07C 313/04

(54) **S-(4-BIPHENYL)-THIOSCHWEFELSÄUREN UND IHRE SALZE, VERFAHREN ZU DEREN HERSTELLUNG UND DIE HERSTELLUNG VON 4-MERCAPTOBIPHENYLEN**
S-(4-BIPHENYL)-THIOSULPHURIC ACIDS AND THEIR SALTS, METHOD FOR PRODUCING THE SAME AND METHOD FOR PRODUCING 4-MERCAPTOBIPHENYLS
ACIDES S-(4-BIPHENYLE)-THIOSULFURIQUES ET LEURS SELS, PROCEDE PERMETTANT DE LES PREPARER ET PREPARATION DE 4-MERCAPTOBIPHENYLES

(30) Priorität: 22.10.1997 DE 19746540
(43) Veröffentlichungstag der Anmeldung: 09.08.2000
(73) Patentinhaber: BAYER AG, 51368 Leverkusen (DE)
(72) Erfinder: ULLRICH, Friedrich-Wilhelm, D-51465 Bergisch-Gladbach (DE); FIEGE, Helmut, D-51373 Leverkusen (DE); EYMANN, Wolfgang, D-51061 Köln (DE)
(86) Internationale Anmeldenummer: EP9806453
(87) Internationale Veröffentlichungsnummer: WO9920604

(56) Entgegenhaltungen:
- GB-A- 1 121 722
- US-A- 2 712 547
- "HOUBEN-WEYL METHODEN DER ORGANISCHEN CHEMIE, 4. Auflage, Band IX" 1955 , GEORGE THIEME VERLAG , STUTTGART, DE XP002094685 siehe Seiten, 18-19, 23-27, 29-33, 67-68, 306-307
- P. BAUMGARTEN: BERICHTE DER DEUTSCHEN CHEMISCHEN GESELLSCHAFT, Bd. 63, 1930, Seiten 1330-1335, XP002094684
- DATABASE CHEMABS CHEMICAL ABSTRACTS SERVICE, COLUMBUS, OHIO, US STN, accession no. 119:138731, XP002094686 & JP 05 070427 A (SUMITOMO SEIKA KK) 23. März 1993

## Beschreibung

Die vorliegende Erfindung betrifft neue S-(4-Biphenyl)-thioschwefelsäuren und deren Salze, ein Verfahren zu deren Herstellung ausgehend von S-(4-Biphenyl)-sulfinsäuren und deren Salzen und die Herstellung von 4-Mercaptobiphenylen aus den S-(4-Biphenyl)-thioschwefelsäuren und ihren Salzen.

4-Mercaptobiphenyle sind wichtige Zwischenprodukte zur Herstellung von pharmazeutischen und agrochemischen Wirkstoffen (siehe z.B. BE-A 887 423, US-A 3 912 757 und WO 96/25 936). Es sind schon einige Verfahren zur Herstellung von 4-Mercaptobiphenylen bekannt geworden, diese sind jedoch alle nicht befriedigend.

So kann man Biphenylsulfochlorid mit amalgamiertem Zink, metallischem Zinn oder Zinn(II)-chlorid reduzieren (siehe z.B. J.A.C.S. 66, 1674 (1944), Chem. Ber. 13, 386 (1880) und Ann. Univ. Marie Curie-Sklodowska, Section Aa, Volume Date 1966 No. 21, 65 bis 83 (1967)). Bei allen diesen Verfahren fallen Schwermetallsalze enthaltende Abwässer an, deren Entsorgung großen Aufwand erfordert.

Man kann auch 4-Aminodiphenyl diazotieren, anschließend mit Kaliumethylxanthat umsetzen und den erhaltenen Thioester verseifen (siehe DE-A 23 17 142, S. 34 bis 35). Hierbei ist wasserlösliches Nickelchlorid einzusetzen, das ebenfalls ins Abwasser gelangt und dort großen Aufwand für die Entsorgung erfordert.

Die Umsetzung von p-Hydroxybiphenyl mit Dimethylthiocarbamoylchlorid, anschließender Newman-Kwart-Umlagerung und abschließender Verseifung (siehe J. Het. Chem. 15, 281 (1978) und WO 96/25 936) liefert 4-Mercaptobiphenyl lediglich in 39 %iger Ausbeute.

Wenn man 4-Brombiphenyl mit Natriummethyl- oder -ethylsulfid umsetzt und den entstehenden Thioether spaltet, so erhält man nur dann gute Ausbeuten an 4-Mercaptobiphenyl (z.B. 96 % d.Th.), wenn man die Handhabung des krebserregenden Hexamethylphosphorsäuretriamids in Kauf nimmt (siehe Tetrahedron Lett. 21, 3099 (1980)). Andere Lösungsmittel, z.B. Dimethylformamid ergeben nur deutlich geringere Ausbeuten an 4-Mercaptobiphenyl (z.B. 67 % d.Th. - siehe Synthesis 9, 751 (1983)).

Die Reaktion von 4-Brombiphenyl mit elementarem Schwefel und die Spaltung des Reaktionsproduktes mit Lithiumaluminiumhydrid zu 4-Mercaptobiphenyl erfordert den Einsatz des schwierig zu handhabenden Lithiumaluminiumhydrids (siehe Tetrahedron Lett. 13, 1283(1972).

Es sind auch von aromatischen Sulfochloriden und aromatischen Disulfiden ausgehende Reduktionsverfahren zur Herstellung von 4-Mercaptobiphenyl bekannt. Die Reduktion mit Wasserstoff und Edelmetallkatalysatoren erfordert jedoch Temperaturen bis 150°C und Drucke bis 150 bar (siehe EP-A 2755), diejenige mit rotem Phosphor und Iod führt zu einem Zwangsanfall von Phosphorsäure und Chlorwasserstoff (siehe Chem. Ber. 99, 375 (1966)) und benötigt den nur schwierig zu handhabenden roten Phosphor.

Schließlich ist bekannt, daß Buntesalze, d.h Salze des Typs R-S-SO₃M (R = organischer Rest, M = einwertiges Metall), in wäßrig-sauren Medien im allgemeinen zu Thiolen hydrolysieren (siehe Angew. Chem. 79, 525 (1967)). Im vorliegenden Fall ergaben sich dabei aber Disulfide.

Es wurden nun S-(4-Biphenyl)-thioschwefelsäuren und Salze davon gefunden, die der Formel (I) entsprechen 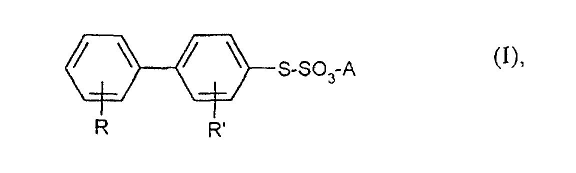 in der
- A: für Wasserstoff, ein Äquivalent eines Metallatoms oder gegebenenfalls substituiertes Ammonium und
- R und R': unabhängig voneinander jeweils für Wasserstoff, C₁-C₆-Alkyl, C₁-C₆-Alkoxy oder Halogen stehen.

In Formel (I) stehen vorzugsweise
- A: für Wasserstoff, Natrium, Kalium, ½ Calcium, ½ Magnesium, ½ Zink, NH₄ oder mit 1 bis 4 C₁-C₆-Alkylresten substituiertes NH₄ und
- R und R': unabhängig voneinander jeweils für Wasserstoff, Methyl, Ethyl, Methoxy, Ethoxy, Fluor oder Chlor.

Besonders bevorzugt stehen in Formel (1)
- A: für Wasserstoff oder Natrium und
- R und R': für Wasserstoff.

Weiterhin wurde ein Verfahren zur Herstellung von S-(4-Biphenyl)-thioschwefelsäuren und deren Salzen der Formel (I) gefunden, das dadurch gekennzeichnet ist, daß man S-(4-Biphenyl)-sulfinsäuren oder deren Salze der Formel 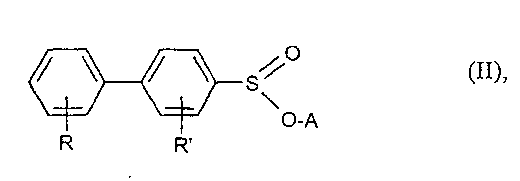 in der
A, R und R' die bei Formel (I) angegebene Bedeutung haben,
mit einer wäßrigen Bisulfitlösung bei einem pH-Wert im Bereich von 2 bis 7 umsetzt.

Die bevorzugten und besonders bevorzugten Bedeutungen von A, R und R' sind auch bei Formel (II) wie bei Formel (I) angegeben.

Die als Ausgangsprodukte benötigten S-(4-Biphenyl)-sulfinsäuren und deren Salze der Formel (II) sind teilweise bekannt und auf bekannte Weise oder analog dazu herstellbar (siehe Ann. Univ. Marie Curie-Sklodowska, Section Aa, Volume Date 1966, No. 21, 49 bis 64 (1967). Die jeweilige Verbindung der Formel (II) muß nicht in reiner Form eingesetzt werden. Sie kann gegebenenfalls z.B. bis zu 25 Gew.-% der entsprechenden Biphenylsulfonsäure und/oder deren Salze enthalten. Vorzugsweise setzt man die jeweilige Verbindung der Formel (II) in Form des Natriumsalzes ein.

Ganz besonders bevorzugt wird das Natriumsalz der S-(4-Biphenyl)-sulfinsäure eingesetzt.

Als wäßrige Bisulfitlösungen können insbesondere wäßrige Alkalibisulfitlösungen eingesetzt werden, wie man sie beispielsweise erhält, wenn man Schwefeldioxid oder schweflige Säure in eine wäßrige Natronlauge oder eine wäßrige Natriumcarbonatlösung einleitet oder Natriumdisulfit (Na₂S₂O₅) in Wasser auflöst. Neben Hydrogensulfitionen kann die wäßrige Bisulfitlösung z.B. zusätzlich Sulfitionen, schweflige Säure oder gelöstes Schwefeldioxid enthalten. Die wäßrige Bisulfitlösung kann z.B. 10 bis 50 gew.-%ig sein, vorzugsweise ist sie 25 bis 45 gew.-%ig. Bezogen auf 1 Mol eingesetzter Verbindung der Formel (II) kann man beispielsweise so viel wäßrige Bisulfitlösung einsetzen, wie 1 bis 5 Molen Bisulfit entspricht. Vorzugsweise liegt diese Menge bei 1,5 bis 3 Molen.

Wenn das Reaktionsgemisch nach dem Zusammenfügen von S-(4-Biphenyl)-sulfinsäure bzw. -salz der Formel (II) und wäßriger Bisulfitlösung einen pH-Wert außerhalb des Bereiches von 2 bis 7 aufweist, ist es noch erforderlich, den pH-Wert in den Bereich 2 bis 7 zu bringen, im einfachsten Fall durch Zugabe von wäßriger Salzsäure.

Vorzugsweise führt man die Umsetzung mit der wäßrigen Bisulfitlösung bei einem pH-Wert von 3 bis 5 durch, der gegebenenfalls durch Säurezugabe, z.B. durch Zugabe von wäßriger Salzsäure, eingestellt werden kann. Je saurer man arbeitet und je länger man die Behandlung in stärker saurem Milieu durchführt je mehr besteht die Gefahr, daß die Reaktion nicht auf der Stufe der S-(4-Biphenyl)-thioschwefelsäuren und deren Salzen der Formel (II) stehenbleibt, sondern bereits die Bildung von entsprechenden Bisdiphenyldisulfiden der Formel (IV) erfolgt (siehe weiter unten). Dies kann erwünscht sein (siehe weiter unten).

Der Umsetzung mit der wäßrigen Bisulfitlösung kann man gegebenenfalls noch Wasser zusetzen, z.B. in einer Menge bis zu 5, vorzugsweise bis zu 3 Gew.-Teilen, bezogen auf einen Gewichtsteil der eingesetzten Verbindung der Formel (II).

Die Umsetzung mit der wäßrigen Bisulfitlösung kann man z.B. bei Temperaturen im Bereich 50 bis 200°C durchführen. Bevorzugt sind 120 bis 170°C. Wenn man bei Temperaturen oberhalb dem Siedepunkt (bei Normaldruck) des Reaktionsgemisches arbeiten will ist der Einsatz von geschlossenen und druckdichten Reaktoren erforderlich. Beispielsweise können in solchen Fällen Drucke bis zu 12 bar auftreten.

Nach Beendigung der Reaktion kann man das erhaltene Salz der S-(4-Biphenyl)-thioschwefelsäure z.B. in roher Form abtrennen, wenn man das Reaktionsgemisch abkühlt, z.B. auf Raumtemperatur, den dann vorliegenden Niederschlag abfiltriert und gegebenenfalls trocknet. Falls gewünscht kann man das Produkt weiter reinigen, z.B. indem man es mit siedendem Ethanol extrahiert.

Aus dem erhaltenen Salz einer S-(4-Biphenyl)-thioschwefelsäure, in der Regel ist es das Natriumsalz, kann man die entsprechende S-(4-Biphenyl)-thioschwefelsäure durch an sich bekannte Methoden freisetzen. Bevorzugt setzt man aus dem Salz einer solchen Thioschwefelsäure die entsprechende Thioschwefelsäure durch Umsetzung an einem sauren Ionenaustauscher frei.

Aus freien S-(4-Biphenyl)-thioschwefelsäuren kann man durch Neutralisation mit einer entsprechenden Base beliebige Salze der jeweiligen S-(4-Biphenyl)-thioschwefelsäure herstellen.

Es wurde auch ein Verfahren zur Herstellung von 4-Mercaptobiphenylen der Formel (III) gefunden 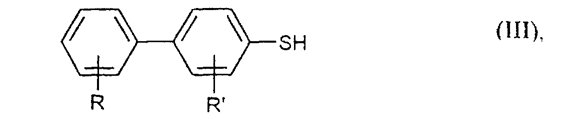 in der
R und R' die bei Formel (I) angegebene Bedeutung haben,
das dadurch gekennzeichnet ist, daß man der Formel (I) entsprechende S-(4-Biphenyl)-thioschwefelsäuren oder deren Salze in Gegenwart einer starken wäßrigen Säure erhitzt und das dabei entstehende Bisdiphenyldisulfid der Formel 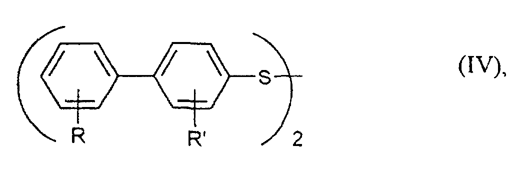 in der
R und R' die bei Formel (I) angegebene Bedeutung haben,
reduziert.

Als starke wäßrige Säuren kann man z.B. Schwefelsäure mit Konzentrationen von beispielsweise 0,05 bis 30, vorzugsweise 1 bis 30 Gew.-% einsetzen. Die Säuremenge kann z.B. so gewählt werden, daß im Reaktionsgemisch ein pH-Wert im Bereich von 0 bis 2 resultiert. Die Temperaturen für die Behandlung mit der starken wäßrigen Säure können z.B. im Bereich 50 bis 200°C, vorzugsweise im Bereich 80 bis 170°C liegen.

Aus dem Reaktionsgemisch kann man das hergestellte Bisdiphenyldisulfid isolieren, indem man das Reaktionsgemisch abkühlt, z.B. auf Raumtemperatur, und den dann vorliegenden Niederschlag abfiltriert und gegebenenfalls trocknet.

Die Reduktion des jeweiligen Bisdiphenyldisulfids der Formel (IV) zum entsprechenden 4-Mercaptobiphenyl der Formel (III) kann beispielsweise nach bekannten Methoden erfolgen wie durch Reduktion mit Natriumborhydrid (siehe DE-A 44 20 777) durch katalytische Reduktion mit Molybdänsulfid-Katalysatoren bei hohen Drucken (siehe J. Org. Chem. 24, 1598 (1959) oder durch katalytische Reduktion mit Palladiumkatalysatoren, Raney-Cobalt oder Raney-Nickel bei hohen Drucken in einem flüssigen 2-Phasensystem (siehe DE-A 17 68 421 und JP-OS 60 199 871).

Bevorzugt führt man diese Reduktion jedoch als katalytische Reduktion mit Metallkatalysatoren der 8. Nebengruppe des PSE oder mit Katalysatorem vom Raney-Typ in alkoholischer Lösung und in Gegenwart alkalisch reagierender Verbindungen durch, beispielsweise bei Temperaturen im Bereich 20 bis 200°C und Drucken bis zu 50 bar. Derartige Reduktionen sind der Gegenstand einer anderen Patentanmeldung, die gleichzeitig vom gleichen Anmelder angemeldet wurde.

Bei der erfindungsgemäßen Herstellung von 4-Mercaptobiphenylen der Formel (III) aus Diphenylsulfinsäuren und deren Salzen der Formel (II) ist es nicht erforderlich, die gebildeten S-(4-Biphenyl)-thioschwefelsäuren und deren Salze der Formel (I) zu isolieren. Man kann bei der Umsetzung mit wäßriger Bisulfitlösung für die Bildung von Bisdiphenyldisulfiden der Formel (IV) geeignete pH-Werte während oder nach der Reaktion einstellen und/oder ausreichend lange Reaktionszeiten anwenden und so ohne Isolierung der S-(4-Biphenyl)-thioschwefelsäuren oder deren Salzen der Formel (I) direkt zum entsprechenden Bisdiphenyldisulfid der Formel (IV) gelangen.

Das jeweilige Bisdiphenyldisulfid der Formel (IV) muß in die Reduktion, insbesondere wenn man diese auf die oben angegebene bevorzugte Weise durchführen möchte, nicht in reiner Form eingesetzt werden. Man kann beispielsweise den bei der Herstellung anfallenden, noch feuchten Filterkuchen mit Alkohol, einer alkalisch reagierenden Verbindung und Katalysator vermischen und danach die Hydrierung durchführen.

Man kann die S-(4-Biphenyl)-sulfinsäuren und deren Salze der Formel (II), die man für die erfindungsgemäße Herstellung von 4-Mercaptobiphenylen der Formel (III) benötigt, auch in situ herstellen, indem man ein entsprechendes Diphenyl-4-sulfochlorid der Formel 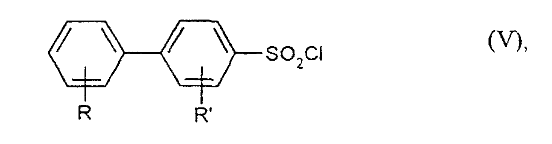 in der
R und R' die bei Formel (I) angegebene Bedeutung haben,
bei einem pH-Wert im Bereich 6 bis 10 mit wäßriger Bisulfitlösung bei 40 bis 80°C umsetzt, danach SO₂ zugibt und auf 70 bis 180°C erhitzt. Man erhält dabei ohne Zwischenisolierung der jeweiligen S-(4-Biphenyl)-sulfinsäuren oder deren Salzen der Formel (II) und ohne Isolierung der jeweiligen S-(4-Biphenyl)-thioschwefelsäuren oder deren Salzen der Formel (I) direkt Bisdiphenyldisulfide der Formel (IV).

Bei dieser Verfahrensweise liegt der pH-Wert bei der Umsetzung mit wäßriger Bisulfitlösung vorzugsweise bei 7,5 bis 9 und die Temperatur nach dem Einleiten von SO₂ vorzugsweise bei 90 bis 160°C. Arbeitet man oberhalb der Siedetemperatur des Reaktionsgemisches (bei Normaldruck) so sind geschlossene und druckfeste Reaktionsgefäße einzusetzen.

Die vorliegende Erfindung stellt ein über die neuen S-(4-Biphenyl)-thioschwefelsäuren oder deren Salze der Formel (I) verlaufendes Verfahren zur Herstellung entsprechender 4-Mercaptobiphenyle der Formel (III) zur Verfügung, das eine Reihe von Vorteilen aufweist. So fallen keine schwermetallhaltigen Abwässer an, die 4-Mercaptobiphenyle der Formel (III) werden in hohen Ausbeuten erhalten, es sind kein besonderer Aufwand zur Handhabung krebserregender Lösungsmittel und keine schwierig zu handhabenden Reagentien erforderlich, es fallen keine Koppelprodukte an und hohe Drucke können vermieden werden. Die Summe dieser positiven Effekte ist ausgesprochen überraschend, da in Angew. Chem. 79 525 (1967) beschrieben ist, daß die saure Hydrolyse von Bunte-Salzen eine allgemein geeignete Methode zur Gewinnung von Thiolen sei. Das ist im vorliegenden Fall nicht so. Unter den beschriebenen Bedingungen wurden hier die Bisdiphenyldisulfide gebildet. Diese müssen reduktiv zu Mercaptan gespalten werden. Es ist insbesondere überraschend, daß trotz der vielen Stufen Biphenylsulfochloride so auf dem Weg über S-(4-Biphenyl)-thioschwefelsäuren in hohen Ausbeuten in 4-Mercaptobiphenyl überführt werden.

### Beispiele

### Beispiel 1

### Herstellung einer Verbindung der Formel (I) aus einer Verbindung der Formel (II)

140 g Diphenylsulfinsäure-Natriumsalz (Gehalt 66,8 Gew.-%, neben 10,5 Gew.-% Diphenylsulfonsäure-Natriumsalz) wurden in 200ml Wasser mit 210ml wäßriger Bisulfitlösung (39 gew.-%ig) verrührt. Mit 37 gew.-%iger wäßriger Salzsäure wurde ein pH-Wert von 4 eingestellt. Diese Mischung wurde in einem Autoklaven auf 150°C erhitzt und dann 2 Stunden bei 150°C und einem Druck zwischen 4,1 und 4,3 bar gerührt. Nach dem Abkühlen auf Raumtemperatur saugte man die entstandene Suspension ab und trocknete den isolierten Feststoff. So wurden 126,4 g rohe S-(4-Biphenyl)-thioschwefelsäure als Natriumsalz mit einer Ausbeute von 78 % d.Th. erhalten. Als Nebenprodukte wurden mittels HPLC außer mitgeschleppter Biphenylsulfonsäure noch unumgesetzte Sulfinsäure festgestellt, aber kein Bis-diphenyl-disulfid. Der isolierte Feststoff wurde mit siedendem Ethanol extrahiert. Beim Abkühlen des Extraktes fiel S-(4-Biphenyl)thioschwefelsäure-Natriumsalz als Monohydrat aus. Das isolierte Produkt wies die für Buntesalze charakteristischen IR-Absorptionsbanden auf. Die Elementaranalyse ergab folgende Erlebnisse (ber./gef.): C 47,1/47,4; H 3,6/3,6; O 20,9/20,0; S 20,9/20,9; Na 7,5/8,0

### Beispiel 2

### Herstellung einer Verbindung der Formel (IV) aus einer Verbindung der Formel (I)

100 ml 10 gew.-%ige wäßrige Schwefelsäure wurden vorgelegt und auf 100°C erhitzt, dann 17 g der Rohsubstanz aus Beispiel 1 zugefügt. Nach kurzer Zeit fiel aus der anfänglich nur schwach getrübten Lösung bei einem pH-Wert von 0 ein weißer Feststoff aus. Nach 30-minütigem Rühren bei 100°C wurde der Ansatz auf Raumtemperatur abgekühlt, dann abgesaugt. Der isolierte Feststoff wurde bei 50°C 200 mbar getrocknet und so 8,2 g Bisdiphenyldisulfid mit einem Gehalt von 86,3 Gew.-% (HPLC) erhalten. S-(4-Biphenyl)-thioschwefelsäure konnte darin nicht mehr nachgewiesen werden.

### Beispiel 3

### Herstellung einer Verbindung der Formel (IV) aus einer Verbindung der Formel (V)

440 ml wäßrige Bisulfitlösung (39 gew.-%ig) und 550 ml Wasser wurden mit 125 ml wäßriger Natronlauge (45 gew.-%ig) vorgelegt und mit 2,2 g Triethylbenzylammoniumchlorid versetzt. Die Mischung wurde auf 60°C erwärmt. Im Laufe einer Stunde wurden 289,5 g Diphenyl-4-sulfochlorid (96 gew.-%ig) eingetragen und gleichzeitig der pH-Wert durch Zudosierung von 45 gew.-%iger wäßriger Natronlauge auf 8 gehalten. Die pH-Kontrolle wurde auch während der 3-stündigen Nachrührzeit durchgeführt. Danach wurde das Reaktionsgemisch in einem Email-Autoklaven mit 80 ml Schwefeldioxid versetzt und 3 Stunden auf 130°C erhitzt. Der Ansatz wurde 6 Stunden bei 130°C und einem Druck zwischen 4,4 und 6,9 bar gerührt. Dabei wurde die Stufe zur S-(4-Biphenyl)-thioschwefelsäure durchlaufen. Anschließend wurde auf Raumtemperatur abgekühlt, entspannt und die erhaltene Suspension abgesaugt. Der Nutschkuchen wurde getrocknet und 237,4 g Bisdiphenyldisulfid mit einem Gehalt von 78,3 Gew.-% erhalten. Das entspricht einer Ausbeute von 91,2 % d.Th.

### Beispiel 4

### Herstellung einer Verbindung der Formel (III) aus einer Verbindung der Formel (IV)

314 g Bisdiphenyldisulfid (erhalten entsprechend Beispiel 3) wurde als feuchter Nutschkuchen mit 11 Ethanol angeschlämmt und mit 44 g Natriumhydroxid und 24,7 g Natriumborhydrid versetzt. Das so erhaltene Gemisch wurde 6 Stunden bei 70°C gerührt dann die entstandene Suspension bei 70°C abgesaugt. Der Filterrückstand wurde mit 250 ml Ethanol gewaschen. Das Filtrat und die Waschflüssigkeit wurden vereinigt und mit 900 ml 7 gew.-%iger wäßriger Salzsäure auf einen pH-Wert von 1 eingestellt. Das ausfallende Produkt wurde abgesaugt, mit 500 ml Wasser gewaschen und getrocknet. Man erhielt 174,2 g eines weißen Pulvers mit einem Schmelzpunkt von 111 bis 113°C. Sein Gehalt an 4-Mercaptobiphenyl lag bei 98,3 Gew.-% (iodometrisch bestimmt). Das entspricht einer Ausbeute von 83,6 % d.Th. bezogen auf eingesetztes Diphenyl-4-sulfochlorid.

### Beispiel 5

### Herstellung einer Verbindung der Formel (III) aus einer Verbindung der Formel (IV)

270 g Bisdiphenyldisulfid wurden wie in Beispiel 3 hergestellt und als feuchter Nutschkuchen mit 800 ml Ethanol angeschlämmt sowie mit 44 g Natriumhydroxid und 5,5 g Raney-Nickel versetzt. Die Mischung wurde in einem Autoklaven auf 80°C erwärmt und dann 10 bar Wasserstoff aufgedrückt. Die Wasserstoffaufnahme war nach 4 Stunden beendet. Der Ansatz wurde gekühlt und entspannt. Nach Absaugen bei 60°C wurde die Mutterlauge mit 840 ml 5,4 gew.-%iger wäßriger Salzsäure angesäuert, wobei das Produkt ausfiel. Es wurde abgesaugt, mit 250 ml Wasser gewaschen und getrocknet. So wurden 178,8 g eines weißen Pulvers mit einem Schmelzpunkt von 110 bis 112°C erhalten. Der Gehalt an 4-Mercaptodiphenyl lag bei 91,8 Gew.-% (iodometrisch bestimmt). Das entspricht einer Ausbeute von 80,1% d.Th., bezogen auf eingesetztes Diphenyl-4-sulfochlorid.

## Patentansprüche

1. S-(4-Biphenyl)-thioschwefelsäuren und deren Salze entsprechend Formel 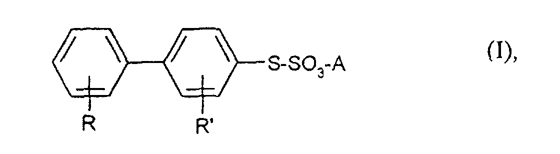 in der
A für Wasserstoff, ein Äquivalent eines Metallatoms oder gegebenenfalls substituiertes Ammonium und
R und R' unabhängig voneinander jeweils für Wasserstoff, C₁-C₆-Alkyl, C₁-C₆-Alkoxy oder Halogen stehen.

2. Säuren und Salze des Anspruchs 1, **dadurch gekennzeichnet, daß** in Formel (I)
A für Wasserstoff, Natrium, Kalium, ½ Calcium, ½ Magnesium, ½ Zink, NH₄ oder mit 1 bis 4 C₁-C₆-Alkylresten substituiertes NH₄ und
R und R' unabhängig voneinander für Wasserstoff, Methyl, Ethyl, Methoxy, Ethoxy, Fluor oder Chlor stehen.

3. Verfahren zur Herstellung von S-(4-Biphenyl)-thioschwefelsäuren und deren Salzen des Anspruchs 1, **dadurch gekennzeichnet, daß** man S-(4-Biphenyl)-sulfinsäuren oder deren Salze der Formel 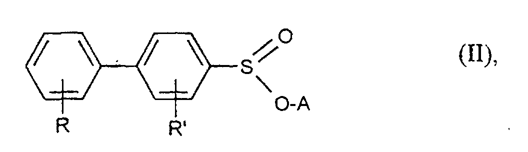 in der
A, R und R' die in Anspruch 1 angegebene Bedeutung haben,
mit einer wäßrigen Bisulfitlösung bei einem pH-Wert im Bereich von 2 bis 7 umsetzt.

4. Verfahren nach Anspruch 3, **dadurch gekennzeichnet, daß** man 1 bis 5 Mole Bisulfit pro Mol der Verbindung der Formel (II) einsetzt und die Umsetzung mit der wäßrigen Bisulfitlösung bei Temperaturen im Bereich 50 bis 200°C vornimmt.

5. Verfahren zur Herstellung von 4-Mercaptobiphenylen der Formel 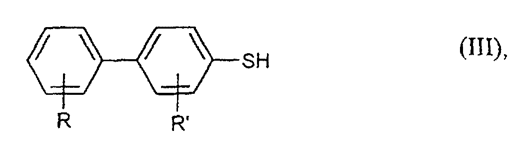 in der
R und R' die in Anspruch 1 angegebene Bedeutung haben,
**dadurch gekennzeichnet, daß** man dem Anspruch 1 entsprechende S-(4-Biphenyl)-thioschwefelsäuren und deren Salze, die man durch die Umsetzung von S-(4-Biphenyl)-sulfinsäuren oder deren Salzen der Formel (II) aus Anspruch 3 mit wäßriger Bisulfitlösung bei pH-Werten von 2 bis 7 erhalten hat in Gegenwart einer starken wäßrigen Säure erhitzt und das dabei entstehende Bisdiphenyldisulfid der Formel 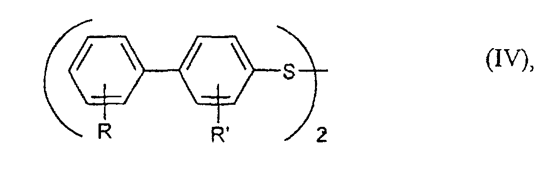 in der
R und R' die in Anspruch 1 angegebene Bedeutung haben,
reduziert.

6. Verfahren nach Anspruch 5, **dadurch gekennzeichnet, daß** man als starke wäßrige Säure 0,05 bis 30 gew.-%ige wäßrige Schwefelsäure einsetzt und im Reaktionsgemisch einen pH-Wert im Bereich von 0 bis 2 einhält.

7. Verfahren nach Ansprüchen 5 und 6, **dadurch gekennzeichnet, daß** man das Erhitzen in Gegenwart einer starken wäßrigen Säure auf 50 bis 200°C durchführt.

8. Verfahren nach Ansprüchen 5 bis 7, **dadurch gekennzeichnet, daß** man die Reduktion mit Natriumborhydrid oder als katalytische Reduktion mit Molybdänsulfid-Katalysatoren, Palladium-Katalysatoren, Raney-Kobalt oder Raney-Nickel durchführt.

9. Verfahren nach Ansprüchen 5 bis 8, **dadurch gekennzeichnet, daß** man die S-(4-Biphenyl)-thioschwefelsäuren und deren Salze der Formel (I) nicht isoliert.

10. Verfahren nach Ansprüchen 5 bis 9, **dadurch gekennzeichnet, daß** man die benötigten S-(4-Biphenyl)-sulfinsäuren und deren Salze der Formel (II) in situ herstellt, indem man ein entsprechendes Diphenyl-4-sulfochlorid der Formel 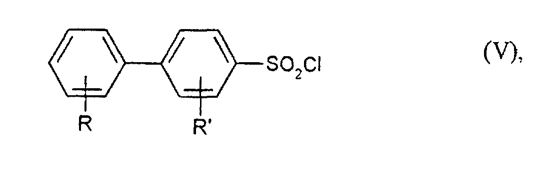 in der
R und R' die in Anspruch 1 angegebenen Bedeutungen haben,
bei einem pH-Wert im Bereich 6 bis 10 mit wäßriger Bisulfitlösung bei 40 bis 80°C umsetzt, danach SO₂ zugibt und auf 70 bis 180°C erhitzt.

## Claims

1. S-(4-biphenyl)-thiosulphuric acids and salts thereof corresponding to the formula 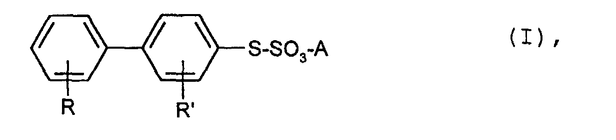 in which
A represents hydrogen, an equivalent of a metal atom or optionally substituted ammonium and
R and R' independently of one another each represent hydrogen, C₁-C₆-alkyl, C₁-C₆-alkoxy or halogen.

2. Acids and salts of Claim 1, **characterized in that** in formula (I)
A represents hydrogen, sodium, potassium, ½ calcium, ½ magnesium, ½ zinc, NH₄ or NH₄ which is substituted by 1 to 4 C₁-C₆-alkyl radicals and
R and R' independently of one another represent hydrogen, methyl, ethyl, methoxy, ethoxy, fluorine or chlorine.

3. Process for preparing S-(4-biphenyl)-thiosulphuric acids and salts thereof of Claim 1, **characterized in that** S-(4-biphenyl)-sulphinic acids or salts thereof of the formula 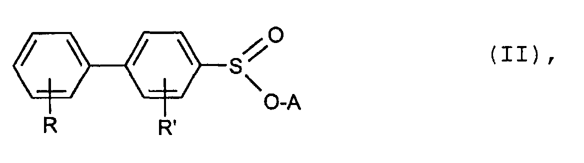 in which
A, R and R' are as defined in Claim 1,
are reacted with an aqueous bisulphite solution at a pH in the range from 2 to 7.

4. Process according to Claim 3, **characterized in that** 1 to 5 mols of bisulphite are employed per mole of the compound of the formula (II) and the reaction with the aqueous bisulphite solution is carried out at temperatures in the range from 50 to 200°C.

5. Process for preparing 4-mercaptobiphenyls of the formula 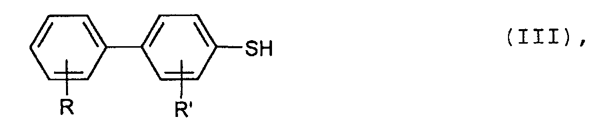 in which
R and R' are as defined in Claim 1,
**characterized in that** S-(4-biphenyl)-thiosulphuric acids and salts thereof corresponding to Claim 1 which were obtained by the reaction of S-(4-biphenyl)-sulphinic acids or salts thereof of the formula (II) from Claim 3 with aqueous bisulphite solution at a pH of from 2 to 7 are heated in the presence of a strong aqueous acid and the resulting bisdiphenyl disulphide of the formula 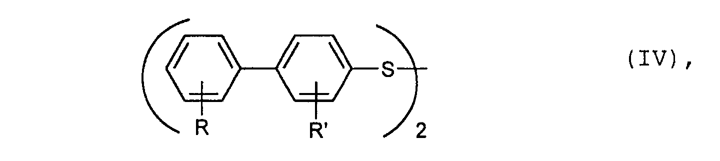 in which
R and R' are as defined in Claim 1,
is reduced.

6. Process according to Claim 5, **characterized in that** the strong aqueous acid used is 0.05 to 30% by weight strength aqueous sulphuric acid and a pH in the range from 0 to 2 is maintained in the reaction mixture.

7. Process according to Claims 5 and 6, **characterized in that** the heating in the presence of a strong aqueous acid is carried out to from 50 to 200°C.

8. Process according to Claims 5 to 7, **characterized in that** the reduction is carried out using sodium borohydride or as a catalytic reduction using molybdenum sulphide catalysts, palladium catalysts, Raney cobalt or Raney nickel.

9. Process according to Claims 5 to 8, **characterized in that** the S-(4-biphenyl)-thiosulphuric acids and the salts thereof of the formula (I) are not isolated.

10. Process according to Claims 5 to 9, **characterized in that** the S-(4-biphenyl)-sulphinic acids and salts thereof of the formula (II) required are prepared in situ by reacting a corresponding diphenyl-4-sulphonyl chloride of the formula 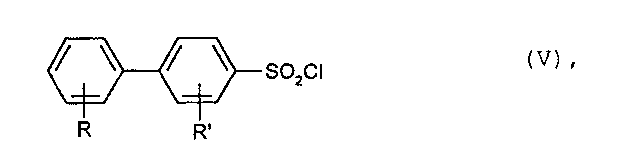 in which
R and R' are as defined in Claim 1,
at a pH in the range from 6 to 10 with aqueous bisulphite solution at from 40 to 80°C, followed by addition of SO₂ and heating to from 70 to 180°C.

## Revendications

1. Acides S-(4-biphényl)thiosulfurique et leurs sels, correspondant à la formule (I) : 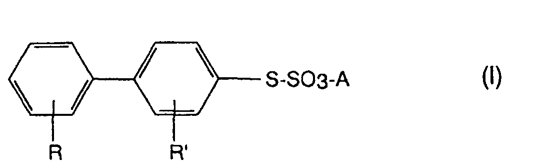 dans laquelle :
A représente l'atome d'hydrogène, un équivalent d'un atome métallique ou un radical ammonium éventuellement substitué, et
R et R' représentent indépendamment l'un de l'autre, chaque fois l'atome d'hydrogène, les radicaux alcoyle en C₁-C₆, alcoxy en C₁-C₆ ou un atome d'halogène.

2. Acides et sels suivant la revendication 1, **caractérisés en ce que** dans la formule (I) :
A représente les atomes d'hydrogène, de sodium, de potassium, ½ calcium, ½ magnésium, ½ zinc, les radicaux NH₄ ou NH₄ substitué par 1 à 4 radicaux alcoyle en C₁-C₆, et
R et R' représentent indépendamment l'un de l'autre, chaque fois l'atome d'hydrogène, les radicaux méthyle, éthyle, méthoxy, éthoxy, les atomes de fluor ou de chlore.

3. Procédé de préparation d'acides S-(4-biphényl)thiosulfuriques et de leurs sels suivant la revendication 1, **caractérisé en ce que** l'on fait réagir des acides S-(4-biphényl)sulfiniques ou leurs sels de formule : 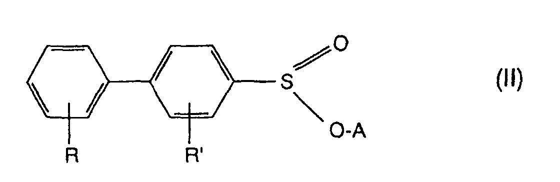 dans laquelle :
A, R et R' ont la signification indiquée à la revendication 1,
avec une solution aqueuse de bisulfite à un pH situé dans l'intervalle allant de 2 à 7.

4. Procédé suivant la revendication 3, **caractérisé en ce que** l'on met en oeuvre 1 à 5 moles de bisulfite par mole du composé de formule (II) et la réaction avec la solution aqueuse de bisulfite se déroule à des températures situées dans l'intervalle allant de 50 à 200°C.

5. Procédé de préparation de 4-mercaptobiphénylènes de formule : 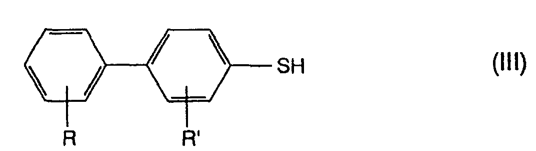 dans laquelle :
R et R' ont la signification donnée à la revendication 1, **caractérisé en ce que** l'on chauffe l'acide S-(4-biphényl)thiosulfurique ou son sel suivant la revendication 1, que l'on obtient par la réaction des acides S-(4-biphényl)sulfiniques ou de leurs sels de formule (II) suivant la revendication 3 avec la solution aqueuse de bisulfite à un pH de 2 à 7, en présence d'un acide fort aqueux et **en ce que** l'on réduit le bisdiphényldisulfure ainsi formé, de formule : 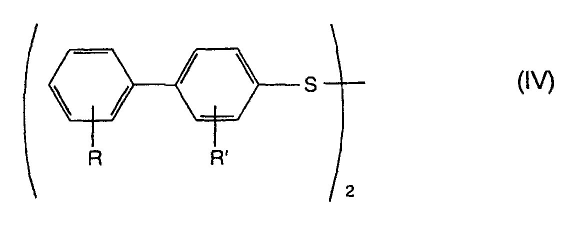
dans laquelle :
R et R' ont la signification donnée à la revendication 1.

6. Procédé suivant la revendication 5, **caractérisé en ce que** l'on met en oeuvre comme acide fort aqueux, de l'acide sulfurique aqueux à 0,05 à 30% en poids et on maintient le mélange réactionnel à un pH dans l'intervalle allant de 0 à 2.

7. Procédé suivant les revendications 5 et 6, **caractérisé en ce que** l'on réalise le chauffage en présence d'un acide fort aqueux à 50 - 200°C.

8. Procédé suivant les revendications 5 à 7, **caractérisé en ce que** l'on réalise la réduction avec du borohydrure de sodium ou par réduction catalytique avec des catalyseurs au sulfure de molybdène, des catalyseurs au palladium, du cobalt de Raney ou du nickel de Raney.

9. Procédé suivant les revendications 5 à 8, **caractérisé en ce que** l'on n'isole pas les acides S-(4-biphényl)thiosulfuriques et leurs sels de formule (I).

10. Procédé suivant les revendications 5 à 9, **caractérisé en ce que** l'on prépare in situ les acides S-(4-biphényl)sulfiniques et leurs sels de formule (II) nécessaires, **en ce que** l'on fait réagir un chlorure de diphényl-4-sulfonyle approprié de formule : 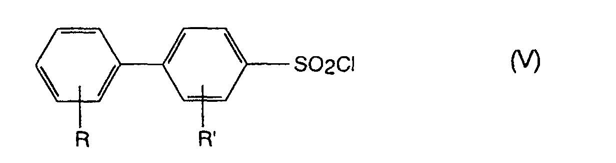 dans laquelle
R et R' ont la signification indiquée à la revendication 1, à un pH situé dans l'intervalle allant de 6 à 10 avec la solution aqueuse de bisulfite à 40-80°C, ensuite on ajoute du SO₂ et on chauffe à 70-180°C.
